# EUROPEAN PATENT SPECIFICATION

(11) **EP 2 198 700 B1**
(45) Date of publication and mention of the grant of the patent: **05.09.2012**
(21) Application number: 08021358.0
(22) Date of filing: 09.12.2008
(51) Int. Cl.: A01H 3/00, A01H 5/00, C12N 15/82

(54) **Method for the production of a transgene-free plant with altered methylation pattern**
Verfahren zur Herstellung einer transgenfreien Pflanze mit verändertem Methylierungsmuster
Procédé de production d'une plante non transgénique avec un motif à méthylation altérée

(43) Date of publication of application: 23.06.2010
(73) Proprietor: RLP AgroScience GmbH, 67435 Neustadt/Weinstrasse (DE)
(72) Inventor: Wassenegger, Michael, Dr. habil., 67435 Neustadt (DE); Krczal, Gabriele, Dr. Prof., 67433 Neustadt (DE); Dalakouras, Athanasios, 67435 Neustadt a.d. Weinstrasse (DE)
(74) Representative: Krauss, Jan

(56) References cited:
- WO-A-2008/143786
- BENDER JUDITH: "DNA methylation and epigenetics" ANNUAL REVIEW OF PLANT BIOLOGY, vol. 55, 2004, pages 41-68, XP009116283 ISSN: 1040-2519
- AUFSATZ WERNER ET AL: "RNA-directed DNA methylation in Arabidopsis." PROCEEDINGS OF THE NATIONAL ACADEMY OF SCIENCES OF THE UNITED STATES OF AMERICA, vol. 99, no. Suppl. 4, 10 December 2002 (2002-12-10), pages 16499-16506, XP009116286 ISSN: 0027-8424
- PICKFORD A S ET AL: "RNA-mediated gene silencing." CMLS CELLULAR AND MOLECULAR LIFE SCIENCES, vol. 60, no. 5, May 2003 (2003-05), pages 871-882, XP009116285 ISSN: 1420-682X
- KINOSHITA YUKI ET AL: "Control of FWA gene silencing in Arabidopsis thaliana by SINE-related direct repeats" PLANT JOURNAL, vol. 49, no. 1, January 2007 (2007-01), pages 38-45, XP009116284 ISSN: 0960-7412
- MATZKE ET AL: "Targets of RNA-directed DNA methylation" CURRENT OPINION IN PLANT BIOLOGY, QUADRANT SUBSCRIPTION SERVICES, GB, vol. 10, no. 5, 4 October 2007 (2007-10-04), pages 512-519, XP022286650 ISSN: 1369-5266
- BOYKO ALEX ET AL: "Epigenetic control of plant stress response" ENVIRONMENTAL AND MOLECULAR MUTAGENESIS, vol. 49, no. 1, January 2008 (2008-01), pages 61-72, XP009116395 ISSN: 0893-6692
- CHUNG KWAN-HO ET AL: "Polycistronic RNA polymerase II expression vectors for RNA interference based on BIC/miR-155" NUCLEIC ACIDS RESEARCH, vol. 34, no. 7, 2006, page Article No.: e53, XP002520392 ISSN: 0305-1048
- SAMAKOGLU SELDA ET AL: "A genetic strategy to treat sickle cell anemia by coregulating globin transgene expression and RNA interference" NATURE BIOTECHNOLOGY, vol. 24, no. 1, January 2006 (2006-01), pages 89-94, XP009119236 ISSN: 1087-0156
- MOLINIER JEAN ET AL: "Transgeneration memory of stress in plants" NATURE (LONDON), vol. 442, no. 7106, August 2006 (2006-08), pages 1046-1049, XP009116336 ISSN: 0028-0836
- LIU HONG-XIANG ET AL: "Effects of secondary structure on pre-mRNA splicing: Hairpins sequestering the 5' but not the 3' splice site inhibit intron processing in Nicotiana plumabinifolia", EMBO (EUROPEAN MOLECULAR BIOLOGY ORGANIZATION) JOURNAL, vol. 14, no. 2, 1995, pages 377-388, ISSN: 0261-4189

## Description

### FIELD OF THE INVENTION

The present invention relates to materials and methods for the production of a transgene-free plant with altered methylation patterns. Particular embodiments of the invention provide methods, nucleic acids, vectors, plant cells, plants and kits, suitable to produce a plant, which is devoid of any transgenic materials, and therefore is transgene-free, but which shows an altered DNA methylation pattern. The present invention furthermore relates to methods, nucleic acids, vectors, plant cells, plants and kits for the production of a transgene-free plant wherein the altered methylation pattern results in the differential expression of a gene. Finally, the present invention also encompasses a method for modifying the methylation pattern in a target DNA sequence of a plant cell.

### DESCRIPTION

To date, the generation of genetically modified organisms (GMO), in particular plants, is conventionally performed by either stably introducing a foreign nucleotide sequence into the genome of the organism or by targeted knock-out or knock-down of a desired part of the genome, e.g. by homologous recombination or RNA interference technologies. However, these approaches result in heritable changes in the DNA sequence of the targeted genome. In most cases, in particular for genetically introduced antibiotic resistances, the farming of such modified plants is very controversially discussed within the public and their release into the environment underlies strict regulations and risk assessments within the European Union and many other countries. Therefore only very few of such genetically engineered plants, or products derived thereof, are currently on the European market. The same holds true for RNA dependent gene silencing mechanisms, primarily post transcriptional gene silencing (PTGS) or RNA directed DNA methylation (RdDM), in which so far the permanent presence of a transgenic construct is necessary to maintain the gene silencing effect in the plant of interest.

WO 02/101059 relates to transgenic plants or plant cells wherein the expression of proteins is altered that are involved in the metabolism of starch. The described methods relate to transgenic constructs that encode RNA species usable for the targeted silencing of the genes encoding these proteins. However, the aforementioned restrictions for the farming of GMOs also apply for the described transgenic plants of the invention.

In view of the above, it is an object of the present invention to provide methods and/or materials that allow for the directed modification of heritable traits in a plant, such as gene expression, but wherein the plant genome sequence remains free of any nucleotide changes, and thereby circumvents the various problems and disadvantages associated with the farming of genetically modified plants.

In one aspect of the present invention, this object is solved by providing a method for modifying the methylation pattern in a target DNA sequence of a plant cell, comprising the steps of, a) providing a polynucleotide suitable for the induction of RNA-directed DNA methylation in said plant cell, said polynucleotide comprising a sequence that is complementary to said target DNA sequence of said plant cell, b) introducing said polynucleotide into said plant cell, c) expressing said polynucleotide in said transformed cell, thereby inducing RNA-directed DNA methylation, and d) determining the methylation pattern of said target DNA sequence in said transformed cells of the plant.

Another preferred aspect then relates to a method for producing a transgene-free plant with an altered methylation pattern in a target DNA sequence thereof. As described herein, the method is based on RNA directed DNA methylation, and essentially comprises the steps of: a) providing a polynucleotide suitable for the induction of RNA-directed DNA methylation in said plant, said polynucleotide comprising a sequence that is complementary to said target DNA sequence. Conventionally, these nucleotides are dsRNA molecules, in particular RNA hairpins which can be easily expressed in plant cells. Step b) of said method comprises the introduction of said polynucleotide into the cells of a plant. Various methodologies of plant transformation are discussed in more detail below. Step c) of said method comprises the expression of said polynucleotides in the transformed cells. The expression of a polynucleotide of the invention in cells of the plant induces RNA-directed DNA methylation of the targeted DNA sequence. In step d), the methylation pattern of said target DNA sequence in said transformed cells of the plant is determined. Means for the analysis of methylation patterns in a given DNA are described below. In step e) a transformed plant that shows an altered methylation pattern in the targeted DNA sequence is selected and then genetically crossed with itself.

In step f), a plant from the progeny of the cross in e) is selected, wherein the selected is characterized **in that** it is devoid of the polynucleotide as introduced according to step b). Thus, the final product of the described method is a plant, wherein a target DNA sequence, either endogenous or transgenic, is cytosine methylated, but importantly, wherein the plant does not contain any traces of the transgenic polynucleotide that were necessary for the induction of the methylation.

RNA mediated silencing mechanisms can interfere with gene expression at different levels: some RNA-directed mechanisms act at a post-transcriptional level through degradation of targeted messenger RNAs. However, dsRNA-derived species can also direct changes in the chromatin structure of DNA regions with which they share sequence identity. For example, plants use such RNA species to lay down cytosine methylation imprints on identical DNA sequences, providing a fundamental mark for the formation of transcriptionally silent heterochromatin. This process is generally referred to as RNA-directed DNA methylation (RdDM).

RdDM is initiated by the presence of double stranded RNA (dsRNA) molecules in the cell nucleus. They potentially trigger the *de novo* methylation of all cytosine bases which are located in DNA regions complementary to the sequence of the RNA double strand. As a consequence, in mammals and in plants, the methylated DNA positions serve as flags for the remodelling of the surrounding chromatin in a way, that dense heterochromatin can be formed at these loci. Due to the dense chromatin environment other proteins are prohibited from contacting the DNA. In particular transcription factors or components of the transcriptional machinery cannot assemble on the methylated promoter sequences and thus no transcription can occur in these regions. In effect, genes that have methylated regulatory sequences are less transcribed and therefore less expressed.

Strikingly, once a symmetric cytosine residue (CpG) is methylated in a plant DNA, enzymes recognise this substrate after DNA replication, namely the DNA methyltransferases, and can methylate the same cytosine position in the daughter strand - none-methylated positions remain none-methylated. In consequence, a given pattern of DNA methylation can be heritable over many generations. Surprisingly, the inventors discovered, that the characteristic heritable epigenetic changes introduced by RdDM with the method of the present invention, can be employed to alter expression of a target gene of a plant, but wherein the plant genomic material remains unmodified.

Molecules which are conventionally used to induce RdDM in plants are so called dsRNA hairpins (hpRNA). An hpRNA can be encoded by a DNA molecule that contains contiguous sense and antisense sequences which are complementary to each other and separated by a short spacer region. When directly cloned downstream of a suitable promoter, these molecules can be expressed in plant cells to produce an RNA that forms an hairpin structure after transcription. In the nucleus the hairpin RNA is assumed to be processed into about 24 nucleotide long dsRNAs, which are suggested to then guide the RdDM machinery to methylate all DNA sequences that are complementary to the RNA fragments.

Hairpin RNAs, when expressed as described above, are substrates not only for the RdDM pathway, but also induce the post transcriptional gene silencing (PTGS) in plants. PTGS is a process wherein dsRNAs are digested into about 21 nucleotide long small interfering RNAs (siRNAs), which guide the RNA induced silencing complex (RISC) to complementary mRNAs in the cytoplasm, thereby mediating their destruction. PTGS and RdDM are both conventionally used in the art to alter the expression of specific target genes. However, only RdDM is heritable over many generations in the absence of the trigger molecule.

The terms "polynucleotide", "oligonucleotide," and "nucleic acid sequence" are used interchangeably herein to refer to a polymeric (two or more monomers) form of nucleotides of any length, either ribonucleotides or deoxyribonucleotides. Although nucleotides are usually joined by phosphodiester linkages, the term also includes polymers containing neutral amide backbone linkages composed of aminoethyl glycine units. The terms are used only to refer to the primary structure of the molecule. Thus, the term includes double stranded and single stranded DNA molecules as above. It will be recognized that such polynucleotides can be modified, for example, by including a label such as a radioactive, fluorescent or other tag, by methylation, by the inclusion of a cap structure, by containing a substitution of one or more of the naturally occurring nucleotides with a nucleotide analogue, by containing an internucleotide modification such as having uncharged linkages (e.g., methyl phosphonates, phosphotriesters, phosphoramidates, carbamates, or the like), by containing a pendant moiety such as a protein (e.g., a nuclease, toxin, antibody, signal peptide, poly-L-lysine, or the like), by containing an intercalator such as acridine or psoralen, by containing a chelator, which can be a metal such as boron, an oxidative metal, or a radioactive metal, by containing an alkylator, or by having a modified linkage (e.g., an alpha anomeric nucleic acid).

Preferred polynucleotide species according to the present invention are selected from ssDNA, dsDNA tDNA, ssRNA, dsRNA, shRNA, siRNA, and mRNA. Preferably, the polynucleotide is a DNA that codes for a dsRNA molecule, preferably a dsRNA-hairpin. DsRNA hairpins are preferably generated by expression of a DNA construct that encodes contiguous sense and anti-sense sequences that are separated by a spacer. Upon transcription of such a construct, the generated ssRNA molecule forms a double strand by base pairing of the sense and antisense sequences.

The term "spacer", when used in the context of the present invention, relates to short polynucleotide sequences with sizes of usually less than 50,and preferably less than 40 and more preferred less than 30 nucleotides, and most preferred leading to a loop size ranging between 3 to 23 nucleotides that, as single stranded molecules, cannot form internal base pairing. Spacers represent the loop of the later hairpin and are generally used in the art for dsRNA hairpin design in order to facilitate the stable formation of the hairpin stem structure.

In a preferred aspect of the invention, polynucleotides are provided as intron-based constructs. Plant introns are generally characterized by an A/T-rich base composition and the presence of a 5' GT- and 3' AG-splice site (Goodall and Filipowicz, 1991). In view of these relatively loose criteria for intron function in plants, the polynucleotides of the present invention can be inserted into almost any position of a functional plant intron. According to Goodall and Filipowicz, 1991, any nucleotide construct of the present invention, that is intended to function as a plant intron, should in total have a minimal length of about 70 base pairs in order to assure the splicing of said construct.

In yet another preferred embodiment, the inventors applied intron sequences according to the invention that are at least about 60%, preferably about 70%, more preferably about 80%, even more preferably about 90%, most preferably 100% homologous to the third intron of *LeRDR1.* Still, any other functional plant intron might be used for the generation of a nucleotide construct according to the invention.

As used herein, the term "homologous" or "homology" denotes structural similarity between two macromolecules, particularly between two polynucleotides, irrespective of whether said similarity is or is not due to shared ancestry. Conventionally, homology denotes the level of sequence identity measured in percent. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Sambrook et al., 1989, supra.

For the expression of the above constructs in a plant or plant cell, the invention preferably embodies that the described polynucleotides are operable linked to a promoter and to a polyadenylation site, wherein said promoter is **characterized in that** it is functional in said cell of said plant. As a promoter in this context, any sequence element is sufficient that induces transcription of the downstream sequence. The minimal requirements of promoters are very well known in the art and many of such promoters are conventionally used for gene expression in plants.

In a preferred embodiment, in step b) of the invention, the transformation of a plant cell with a polynucleotide as described above, the preferred method is selected from standard procedures known in the art. Transformation of plant tissue can be achieved preferably by particle bombardment (Klein et al., "High- Velocity Microprojectiles for Delivering Nucleic Acids Into Living Cells," Nature 327:70-73 (1987)), also known as ballistic transformation of the host cell, as disclosed in U.S. Patent Nos. 4,945,050, 5,036,006, and 5,100,792, all to Sanford et al., and in Emerschad et al., "Somatic Embryogenesis and Plant Development from Immature Zygotic Embryos of Seedless Grapes (Vitis vinif era) " Plant Cell Reports 14:6-12 (1995). In particle bombardment, tungsten or gold microparticles (1 to 2 µm in diameter) are coated with the DNA of interest and then bombarded at the tissue using high pressure gas. In this way, it is possible to deliver foreign nucleotides into the nucleus. Biologically active particles (e.g., dried bacterial cells containing the vector and heterologous DNA) can also be propelled into plant cells. Other variations of particle bombardment, now known or hereafter developed, can also be used. Another preferred method of stably introducing the nucleic acid construct into plant cells is to infect a plant cell with *Agrobacterium tumefaciens* or *Agrobacterium rhizogenes* previously transformed with the polynucleotide construct. As described above, the Ti (or RI) plasmid of *Agrobacterium* enables the highly successful transfer of a foreign nucleic acid molecule into plant cells. A preferred variation of *Agrobacterium* transformation uses vacuum infiltration in which whole plants are used (Senior, "Uses of Plant Gene Silencing," Biotechnology and Genetic Engineering Reviews 15:79-119 (1998)). Yet another referred method of introduction is fusion of protoplasts with other entities, either mini-cells, cells, lysosomes, or other fusible lipid-surfaced bodies (Fraley et al., Proc. Natl. Acad. Sci. USA 79:1859-63 (1982),). Also preferred in a method, wherein the nucleic acid molecule is introduced into the plant cells by electroporation (Fromm et al., Proc. Natl. Acad. Sci. USA 82:5824 (1985)). In this technique, plant protoplasts are electroporated in the presence of plasmids containing the expression cassette. Electrical impulses of high field strength reversibly permeabilize biomembranes allowing the introduction of the plasmids. Electroporated plant protoplasts reform the cell wall, divide, and regenerate. Other preferred methods of transformation include chemical-mediated plant transformation, microinjection, physical abrasives, viral transduction and laser beams (Senior, "Uses of Plant Gene Silencing," Biotechnology and Genetic Engineering Reviews 15 :79- 119 (1998)). The precise method of transformation is not critical to the practice of the present invention. Any method that results in efficient transformation of the host cell of choice is appropriate for practicing the present invention.

In a preferred embodiment of the invention, Step d) of the inventive method described herein, comprises the analysis of the methylation pattern of the targeted sequence. Preferred for the invention is the most frequently used method for analysing DNA for the presence of 5-methylcytosine, which is based upon the specific reaction of bisulfite with cytosine, whereby, upon subsequent alkaline hydrolysis, cytosine is converted to uracil which corresponds to thymine in its base pairing behaviour. Significantly, however, 5-methylcytosine remains unmodified under these conditions. Consequently, the original DNA is converted in such a manner that methylcytosine, which originally could not be distinguished from cytosine by its hybridization behaviour, can now be detected as the only remaining cytosine using standard, artrecognized molecular biological techniques, for example, by amplification and hybridization, or by sequencing. All of these techniques are based on differential base pairing properties, which can now be fully exploited. Yet another preferred technique to determine the methylation status of a given sequence of the invention, is the use of methylation sensitive restriction endonucleases. These enzymes are able to discriminate between recognition sites that contain methylated bases and therefore either cut or do not cut at the site. The analysis of the digestion pattern of a DNA molecule by southern blots, therefore gives information about the methylation status at the given recognition sites.

In the methods of the present invention, genetic crosses are performed by self-pollination or cross-breeding according to standard techniques established in classical breeding. For example, traditional crossbreeding methods include selecting and sowing seeds from plants with desirable traits.

Further, in a preferred aspect of the invention, step f) comprises an analysing step to determine the presence of any transgenic nucleotides in the selected progeny plant. The preferred standard methodology includes polymerase chain reaction (PCR) analysis of the genomic DNA of the plant. Using specific primers for the introduced polynucleotide, amplification of a PCR product should occur only if the transgenic material is present in the analysed plant. Thus, the person skilled in the art can easily identify individual plants which are devoid of any transgenic molecules.

Preferably, in still another aspect of the invention, the plant DNA sequence targeted for methylation is, but is not limited to, an endogenous regulatory sequence that regulates plant DNA transcription. As used herein, the term "regulatory sequence" means a nucleotide sequence that, when operatively linked to a coding region of a gene, affects transcription of the coding region such, that a ribonucleic acid (RNA) molecule is transcribed from the coding region. A regulatory element generally can increase or decrease the amount of transcription of a nucleotide sequence, for example, a coding sequence, operatively linked to the element with respect to the level at which the nucleotide sequence would be transcribed absent the regulatory element. Regulatory elements are well known in the art and preferably include promoters, enhancers, silencers, inactivated silencer intron sequences, 3'-untranslated or 5'-untranslated sequences of transcribed sequence, preferably a poly-A signal sequence, or other protein or RNA stabilizing elements, insulators which restrict the regulatory effect of these sequences to defined regions, or other gene expression control elements known to regulate gene expression or the amount of expression of a gene product. A regulatory element can be isolated from a naturally occurring genomic DNA sequence or can be synthetic, for example, a synthetic promoter.

The term "gene" as used in the context of the invention describes any DNA sequence element that can be transcribed into RNA and that might encode for a heritable trait in an organism. Most genes are protein coding genes, wherein the nucleotide sequence of the gene codes for the amino acid sequence of the protein product. However, other genes might code for RNAs which are not translated into proteins - so called non-coding RNAs (ncRNAs). For example, ncRNA genes encode for transfer RNAs (tRNAs), or structural RNAs as found in large protein complexes like the ribosome (rRNAs). Further, the term "gene" includes coding regions for small none-coding RNAs species. Small non-coding RNA genes include snoRNAs, microRNAs, siRNAs and piRNAs and long ncRNAs that include examples such as Xist and HOTAIR.

In a preferred embodiment, if the described invention is used to methylate a regulatory sequence in a plant, the method preferably comprises a step for testing the activity of said regulatory sequence in the transformed plant. Conventionally, the activity of a regulatory sequence can be tested by means of quantifying the amount of transcribed RNA of the gene that is under control of said regulatory sequence. To detect the amount of transcripts, the preferred method is selected from Northern blot analysis, quantitative real time reverse transcriptase PCR or run-off transcription analysis.

In still another preferred aspect, the invention can be used to alter the expression of a target gene via methylation of a regulatory sequence that is functionally connected to the gene. In this case, the method preferably comprises an additional step g), determining the gene expression for said target gene. Among others, means as described above for the analysis of the activity of regulatory sequences also can be applied to check gene expression of the target gene. It might be noteworthy that preferred embodiments of the invention comprise methods of altering the expression of a target gene, either directly by targeted methylation of the regulatory sequences of said target gene or indirectly by targeted methylation of regulatory sequences of a factor, e.g. a transcription factor, regulating the expression of said target gene.

Disclosed herein is a transgene-free plant comprising an altered methylation pattern and/or expression pattern, produced according to a method as described herein. Preferably, a plant is selected from variety of plants, including but not limited to, corn (*Zea mays*), Brassica sp. (e.g., *B. napus,* B. rapa, *B. juncea*), alfalfa (*Medicago sativa*), rye (*Secale cereale*), sorghum (*Sorghum bicolor, Sorghum vulgare*), sunflower (*Helianthus annuus*), safflower (Carthamus tinctorius), wheat (*Triticum aestivum*), soybean (Glycine max), tobacco (Nicotiana tabacum), potato (*Solanum tuberosum*), peanuts (*Rachis hypogaea*), cotton (Gossypium barbadense, Gossypium hirsutum), sweet potato (*Ipomoea batatus*), cassava (Manihot esculenta), coffee (Cofea spp.), coconut (*Cocos nucifera*), pineapple (Ananas comosus), citrus trees (*Citrus spp*.), cocoa (Theobroma cacao), tea (Camellia sinensis), banana (Musa spp.), avocado (Perseaultilane), fig (Ficuscasica), guava(Psidium guava), mango (Mangifera indica), olive (Olea europaea), papaya (Carica papaya), cashew(Anacardium occidentale), macadamia (Macadamia integrifolia),ahnond (Prunus amygdalus), sugar beets (Beta vulgaris), sugarcane (Saccharum spp.), oats, duckweed (Lemna), barley, tomatoes (Lycopersicon esculentum), lettuce (e. g., Lactuca sativa), green beans (Phaseolus vulgaris), lima beans (Phaseoluslimensis), peas (Lathyrus spp.), and members of the genus Cucumis such as cucumber (C sativus), cantaloupe (C. cantalupensis), and musk melon (C. melo). Ornamentals such as azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum are also included. Additional ornamentals within the scope of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be employed in practicing the present invention include, for example, pines such as loblolly pine (Pinus taeda), slash pine (Pinus elliotii), ponderosa pine (Pinus ponderosa), lodgepole pine (Pinus contorta), and Monterey pine (Pinus radiata), Douglas-fir (Pseudotsuga menziesii); Western hemlock (Tsugaultilane); Sitka spruce (Picea glauca); redwood (Sequoia sempervirens); true firs such as silver fir (Abies amabilis) and balsam fir (Abies balsamea); and cedars such as Western red cedar (Thuja plicata), and Alaska yellow-cedar (Chamaecyparis nootkatensis).

Also provided are polynucleotide constructs usable for the methylation of a target DNA sequence in a plant cell. A preferred nucleotide construct comprises at least one plant intron, **characterized in that** at least one targeting domain is inserted into said plant intron, wherein said targeting domain, after splicing of said intron, forms a dsRNA that is complementary to said target DNA sequence. The term "targeting domain" as used herein relates to a nucleotide sequence that is used to provide a dsRNA for the induction of RdDM in the nucleus of a plant cell, and that contains, at least in part, sequences that are complementary to the DNA sequence that is targeted for methylation. The description provides such a polynucleotide construct with a targeting domain, wherein the dsRNA is encoded by contiguous sense and anti-sense sequences that are separated by a spacer.

Provided is a vector comprising a nucleic acid according to the present invention. A vector within the meaning of the present description a protein or a nucleic acid or a mixture thereof which is capable of being introduced or of introducing the polynucleotides comprised into a cell. It is preferred that the proteins encoded by the introduced nucleic acid are expressed within the cell upon introduction of the vector.

Preferably, the vector comprises recombinant vectors, plasmids, phagemids, phages, cosmids, viruses, in particular but not limited to virus-derived amplicon vectors, potato virus X based vectors, tobacco rattle virus based vectors, geminivirus-based vectors such as cabbage leaf curl virus and barley stripe mosaic virus based vectors and vectors based on satellite viruses (reviewed in Curtin, S.J., Wang, M.-B., Watson, J.M., Roffey, P., Blanchard, C.L. and Waterhouse, P.M.. (2007), chapter 12, p 291-332 in "Rice Functional Genomics; Challenges, Progress and Prospects". Upadhyaya, Narayana M. (Ed.), ISBN: 978-0-387-48903-2), virosomes, and nucleic acid coated particles, in particular gold spheres.

The term "recombinant nucleic acid molecule" refers to a polynucleotide produced by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise a nucleotide sequence and a regulatory element. A recombinant nucleic acid molecule also can be based on, but different, from a naturally occurring polynucleotide, for example, a polynucleotide having one or more nucleotide changes such that a first codon, which normally is found in the polynucleotide, is replaced with a degenerate codon that encodes the same or a conservative amino acid, or such that a sequence of interest is introduced into the polynucleotide, for example, a restriction endonuclease recognition site or a splice site, a promoter, a DNA replication initiation site, or the like.

Preferred is a recombinant vector, which is an expression vector, optionally comprising one or more genes to be expressed. Preferably, said expression is driven by a regulatory sequence (or sequences). A regulatory sequence can be isolated from a naturally occurring genomic DNA sequence or can be synthetic, for example, a synthetic promoter.

Regulatory sequences can be constitutively expressed regulatory sequences, which maintain gene expression at a relative level of activity (basal level), or can be regulated regulatory sequences. Constitutively expressed regulatory sequence can be expressed in any cell type, or can be tissue specific, which are expressed only in particular cell types, phase specific, which are expressed only during particular developmental or growth stages of a plant cell, or the like. A regulatory sequence such as a tissue specific or phase specific regulatory sequences or an inducible regulatory sequence useful in constructing a recombinant polynucleotide or in a practicing a method of the invention can be a regulatory sequence that generally, in nature, is found in a plant genome. However, the regulatory sequence also can be from an organism other than a plant, including, for example, from a plant virus, an animal virus, or a cell from an animal or other multicellular organism.

A preferred regulatory sequence useful for expression of polynucleotides is a promoter element. Useful promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. A bacterial promoter can be induced to varying levels of gene expression depending on the level of isothiopropyl galactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

The choice of promoter will vary depending on the temporal and spatial requirements for expression, and also depending on the target species. In some cases, expression in multiple tissues is desirable. While in others, tissue-specific, e.g., leaf-specific, seed-specific, petal-specific, anther-specific, or pith-specific, expression is desirable. Although many promoters from dicotyledons have been shown to be operational in monocotyledons and vice versa, ideally dicotyledonous promoters are selected for expression in dicotyledons, and monocotyledonous promoters for expression in monocotyledons. There is, however, no restriction to the origin or source of a selected promoter. It is sufficient that the promoters are operational in driving the expression of a desired nucleotide sequence in the particular cell.

Other sequences that have been found to enhance gene expression in transgenic plants include intron sequences (e. g., from Adh 1, bronze 1, actin 1, actin 2 (WO 00/760067), or the sucrose synthase intron), poly adenylation signals in the 3' prime UTR and viral leader sequences (e.g., from TMV, MCMV and AMV). For example, a number of non-translated leader sequences derived from viruses are known to enhance expression. Specifically, leader sequences from tobacco mosaic virus (TMV), maize chlorotic mottle virus (MCMV), and alfalfa mosaic virus (AMV) have been shown to be effective in enhancing expression (e.g., Gallie et al., 1987; Skuzeski et al., 1990). Other leaders known in the art include but are not limited topicomavirus leaders, for example, EMCV leader (encephalomyocarditis virus 5'-non-coding region; Elroy-Stein et al., 1989); potyvirus leaders, for example, TEV leader (tobacco etch virus); MDMV leader (maize dwarf mosaic virus); human immunoglobulin heavy chain binding protein (BiP) leader, (Macejak et al., 1991); untranslated leader from the coat protein mRNA of AMV (AMV RNA 4; Jobling et al., 1987), TMV (Gallie et al.,1989), and MCMV (Lommel et al., 1991; see also, della Cioppa et al., 1987).

The regeneration, development and cultivation of plants from single plant protoplast, transformants or from various transformed explants is well known in the art (Weissbach and Weissbach, In: Methods for Plant Molecular Biology, Academic Press, San Diego, CA, (1988 )). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil.

Further disclosed is a kit for producing a transgene free plant with an altered methylation pattern and/or expression profile. The kit might include, but is not limited to, materials of the invention selected from polynucleotides, vectors, plasmids, cells, plants and/or parts thereof.

In yet another aspect, plants of the present invention can be used for the production of seeds, fruits, stems, roots and leaves, or other plant derived products, with altered expression of a gene. Preferably, wherein the gene with reduced expression encodes for, or influences an unwanted trait, such as the onset of over-ripeness, or plant products that are harmful for other organisms, particularly for animals, plants or humans. Examples not only include toxic secondary metabolites, e. g. alkaloids (nicotine, saponins) but also allergens or genes influencing the level of poisonous biochemical substances in a given plant. "Allergens" preferably comprise substances which cause an allergic reaction such as asthma, allergic rhinitis, pollinosis, or atopic dermatitis upon contact of a human or an animal with such substance. Preferably included are inhalent allergens from grass or tree pollens. Weeds, grasses and trees are common sources of pollen, and high concentrations of these pollen allergens in the air surrounding us correspond well to pollen-related hypersensitivity disease. These allergenic proteins in pollens with molecular weight about 30 kD are quickly and profusely released by grass pollen upon hydration. Also preferably included are ingestent allergens, which often refer to substances inducing allergy after the sensitized individuals eating a certain food, particularly peanut and/or tree nut allergy. Still another preferred aspect is directed to contact allergens, wherein latex is the most important contact allergen in plants.

Further comprised are substances that influence the taste or smell of the plant or parts derived thereof; or that lead to a certain physical appearance of the plant, e.g. the colour of the plant or physical manifestations like plant height. Further included in the scope of the invention is a use as described above for the production of plants with reduced expression of genes coding for, or influencing enzymes, important in plant biochemical processes.

The present invention will now be further described in the following examples with reference to the accompanying figures and sequences, nevertheless, without being limited thereto. In the Figures and Sequences,

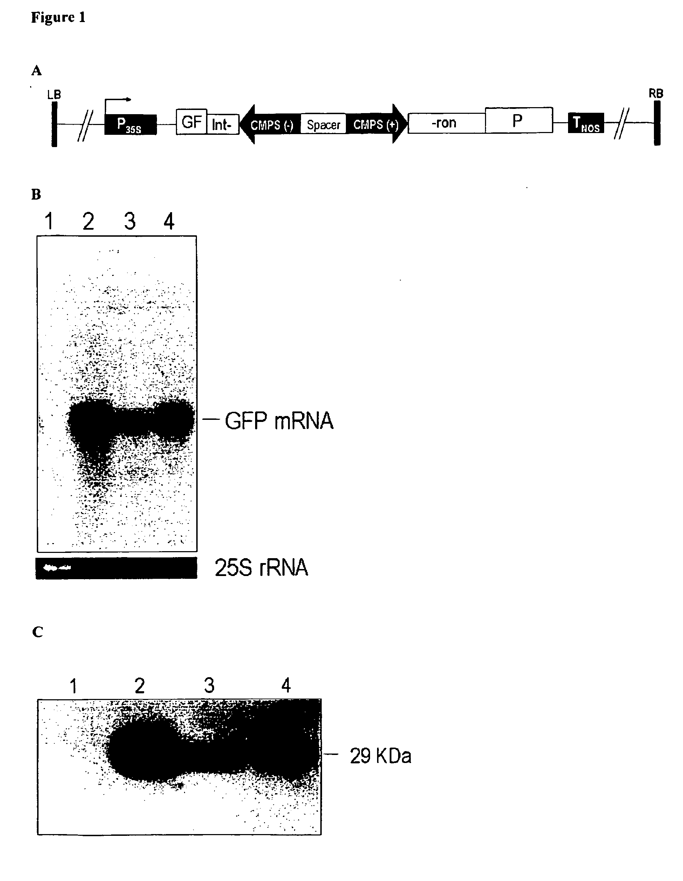

Figure 1 shows the int-hpCMPS construct and its molecular behaviour in planta. (A) Schematic representation of the int-hpCMPS construct. The 634 bp intron 3 of LeRDR1 was introduced inside a 796 bp mGFP5 cDNA unit. Next, the 632 bp hpCMPS was inserted inside the intron. The 35S promoter and NOS terminator, as well as thr right (BR) and left (BL) border of the T-DNA are indicated. The scheme sizes do not correspond to the actual sizes of the fragments. (B) Northern blot, GFP mRNA levels after splicing. Lane 1, SR1 WT; lane 2 and 3, high and low expressing SR1 int-hpCMPS lines respectively; line 4, SR1 GFP15E. The GFP mRNA is of the correct size after splicing, comparing to the positive control SR1 GFP15E (transgenic tobacco without intron in the GFP). The absence of higher molecular weight bands indicate the absence of unspliced pre-mRNA molecules. The probe used was the full length cDNA of GFP. The 25S rRNA below the blot indicate the equal loading of samples (10 µg total RNA). (C) Western blot, GFP protein levels after splicing. Lane 1, SR1 WT; lane 2 and 3, high and low expressing SR1 int-hpCMPS lines respectively; line 4, SR1 GFP15E. The GFP protein was of the correct size (29 KDa), compared to a known GFP transgenic tobacco without intron in GFP. GFP antibody was used for detection.

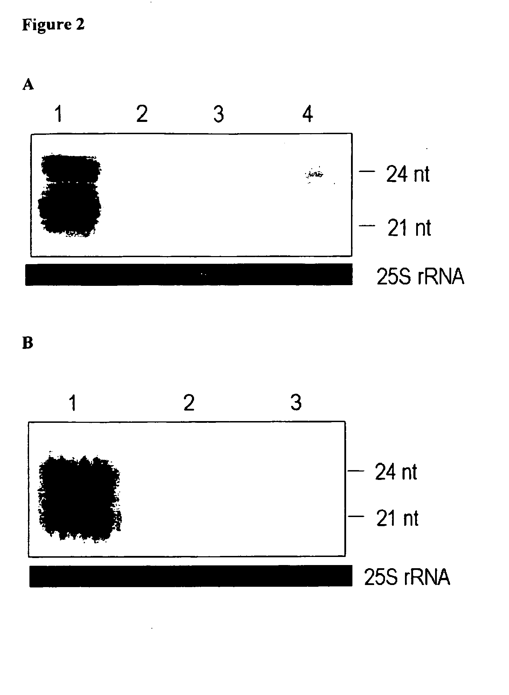

Figure 2 shows siRNA Northern blot analysis in the SR1 int-hpCMPS. (A) Northern blot, CMPS siRNAs in SR1 inthpCMPS. Lane 1, marker; lane 2, SR1 WT; lanes 3 and 4, SR1 int-hpCMPS, low and high expressing respectively. Only 24-nt CMPS siRNAs were detectable and only in the high expressing line (lane 4). 21-nt siRNAs were not detectable. The 632 bp hpCMPS DNA was used as a probe. The 25S rRNA is used as a loading control. Northern blot, GFP siRNAs. (B) Lane 1, SR1 int-hpCMPS agroinfiltrated with a hairpin GFP construct (pCV702SM GpG); lane 2, SR1 int-hpCMPS; lane 3, SR1 WT. In lane 1, 21 and 24 GFP siRNAs were observed, meaning that 21-nt siRNA producing machinery was not impaired in SR1 int-hpCMPS (where only 24-nt CMPS siRNAs were observed). No GFP siRNAs of any size were detected in non agroinfiltrated SR1 int-hpCMPS, meaning that GFP mRNA after splicing is stable and no transitivity takes place. The full length GFP cDNA was used as probe. The 25S rRNA is used as a loading control.

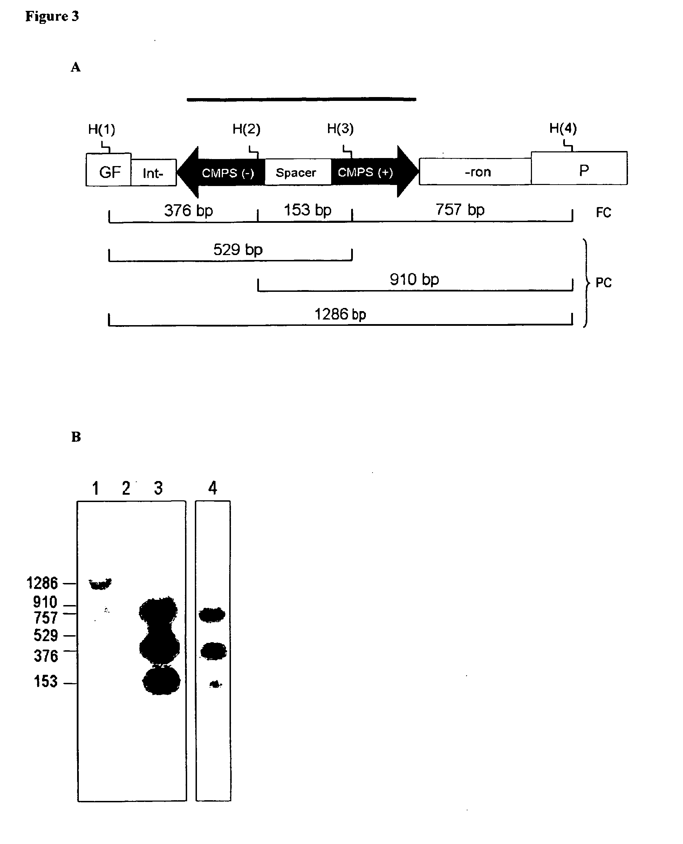

Figure 3 shows the methylation analysis of int-hpCMPS transgene by Southern blot. (A) Schematic representation of the int-hpCMPS transgene and the fragments expected to emerge after full and partial cleavage (FC and PC respectively) with HaeIII, which is blocked when its internal cytosine is methylated. The black bar indicates the probe used. (B) Southern blot, methylation analysis. 20 µg DNA were digested with DraI+HaeIII and analyzed in 1% agarose gel (DraI has no restriction site in the transgene). Lane 1, SR1 int-hpCMPS; lane 2, SR1 WT; lane 3 SR1 WT spiked with some pg of plasmid pPCV702SM int-hpCMPS; lane 4, lighter exposure of lane 3. In plasmid (lane 3), only the FC products light up (757 bp, 376 bp and 153 bp). However, in SR1 int-hpCMPS (lane 1), where methylation takes place in the hairpin, all the PC fragments were noticed (1286 bp, 910 bp, 529 bp) in addition to the FC ones.

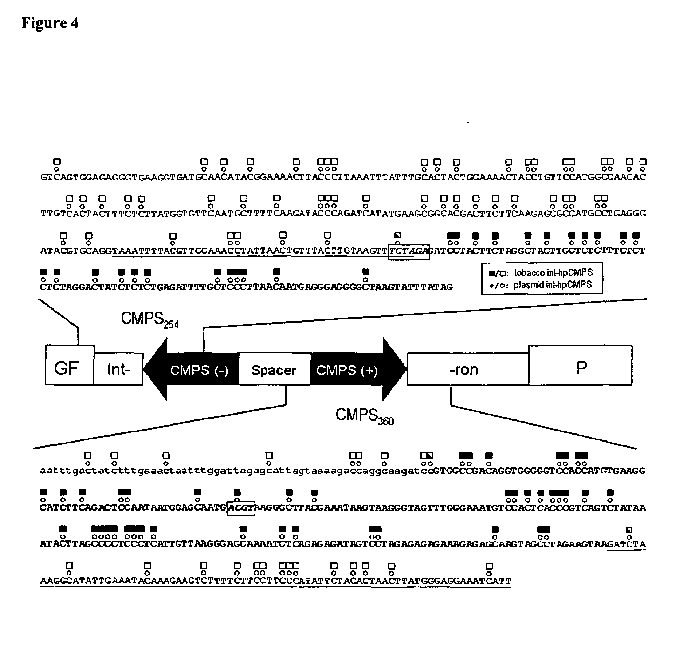

Figure 4 shows the methylation analysis of int-hpCMPS transgene by bisulfite sequencing. Two fragments of the transgene were analyzed separately. The first one (CMPS254) consisted of a part of GFP 5', intron and part of CMPS antisense. The second one (CMPS360) consisted of part of the spacer, of CMPS sense and part of the intron. Above each sequence is represented the methylation status of cytosines in SR1 int-hpCMPS (indicated with squares), while below them, in SR1 WT spiked with plasmid pPCV702SM int-hpCMPS (indicated with circles). The empty square/circle corresponds to 0-10% methylation of cytosines, the half-filled to 50-60 % methylation while the filled to 90-100% methylation. This percentage is based upon 10 independent clones analyzed. The intronic sequences are underlined, the CMPS sequences are in bold and the spacer sequence in small letters.. The 5'-TCTAGA-3' XbaI and 5'-ACGT-3' HpyCH4IV restriction sites are indicated as sequence in a frame. In both, 254 bp and 360 bp parts, methylation was observed strictly to CMPS sequence to almost 100% (filled squares) and it did not spread to flanking intronic or GFP sequences (empty squares). The plasmid control indicated a full conversion of cytosines in bisulfite reaction (empty circles).

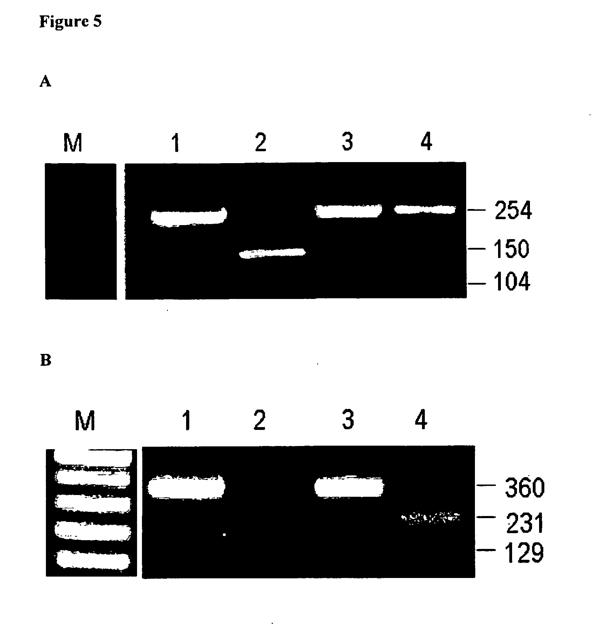

Figure 5 shows the validation of bisulfite data by restriction digests of the PCR products with XbaI and HpyCHIV4. (A) The CMPS259 PCR product was challenged with XbaI (see Figure 4, sequence in frame), which separated it in a 150 bp and 104 bp products. Lane 1, CMPS259 amplicon from untreated and undigested plasmid; lane 2, CMPS259 amplicon from untreated and XbaI digested plasmid; lane 3 CMPS259 amplicon from bisulfite treated and XbaI digested plasmid; lane 4 CMPS259 amplicon from bisulfite treated and XbaI digested tobacco. CMPS259 amplicon from untreated plasmid was almost 100% cleaved (lane 2), while from bisulfite treated plasmid (lane 3) was not cleaved at all, indicating resistance to XbaI due to deamination of cytosines to thymines and destruction of restriction site. In the case of CMPS259 amplicon obtained from plant, where almost 50% of cytosines are methylated (Figure 4), only the 50% of the amplicons were cleaved, in validation of the bisulfite data. (B) The CMPS360 PCR product, was challenged with HpyCH4IV (see Figure 4, sequence in frame), which separated it in 231 bp and 129 bp. Lane 1, CMPS360 amplicon from untreated and undigested plasmid; lane 2, CMPS360 amplicon from untreated and HpyCHIV4 digested plasmid; lane 3 CMPS360 amplicon from bisulfite treated and HpyCHIV4 digested plasmid; lane 4 CMPS360 amplicon from bisulfite treated and HpyCHIV4 digested tobacco. CMPS360 amplicon from untreated plasmid was almost 100% cleaved (lane 2), while from bisulfite treated plasmid (lane 3) was not cleaved at all, indicating resistance to HpyCHIV4 due to deamination of cytosines to thymines and destruction of restriction site. In the case of CMPS360 amplicon obtained from plant, where almost 100% of cytosines are methylated (Figure 4), almost 100% of the amplicon was cleaved, in validation of the bisulfite data.

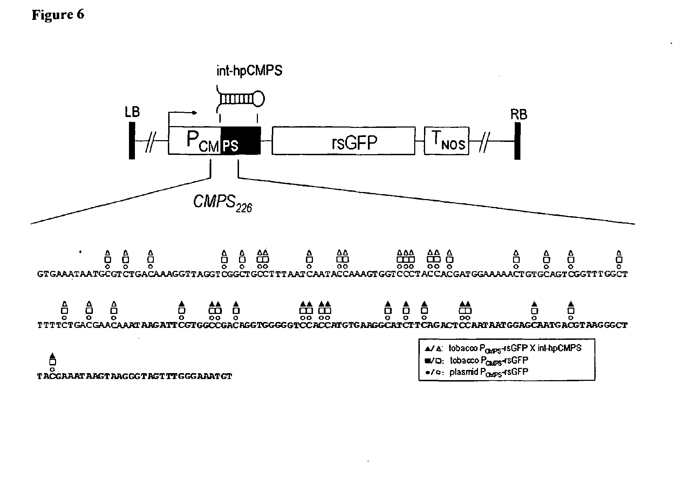

Figure 6 shows the bisulfite sequencing for the ability of the int-hpCMPS to trigger in trans RdDM of the sensor construct P_{CMPS}-rsGFP. In the sensor construct, the rsGFP cDNA (791 bp) is transcribed from the CmYLCV CMPS promoter (406 bp). The overlapping part of the promoter with the sequence of CMPS used in hpCMPS is indicated with black colour (240 bp). This is the region where in trans methylation is expected. A 226 bp fragment (CMPS226) with a overlapping and non-overlapping region with the methylation trigger molecule int-hpCMPS was analyzed by bisulfite sequencing. The methylation status is indicated above the sequence, for plasmid (circles), SR1 PCMPS-rsGFP (squares) and SR1 PCMPS-rsGFP X SR1 int-hpCMPS (triangle). The empty circle/square/triangle corresponds to 0-10% methylation of cytosines, the half-filled to 50-60 % methylation while the filled to 90-100% methylation. This percentage is based upon 10 independent clones analyzed. While the PCMPS under analysis of plasmid or plant prior to crossing with the trigger int-hpCMPS was fully converted', after the crossing, the part that overlapped with the trigger hpCMPS (bold sequence) was fully methylated and this methylation did not spread to non overlapping region of PCMPS.

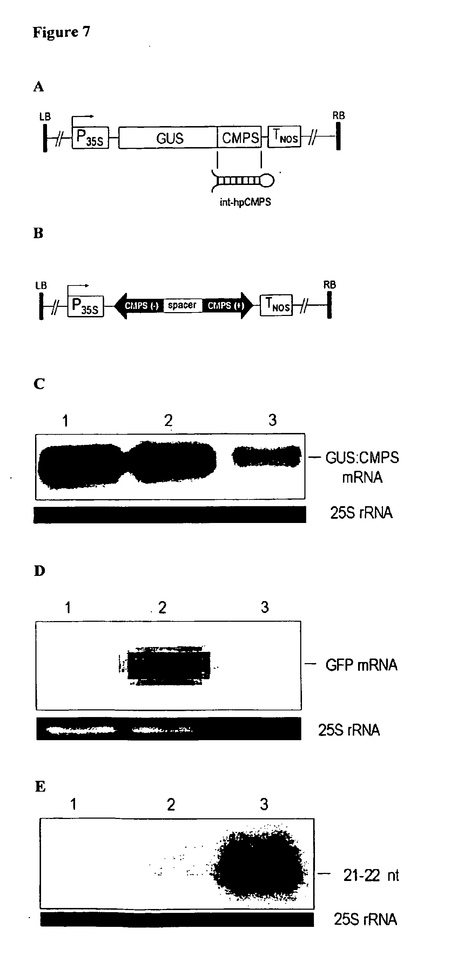

Figure 7 shows the analysis of the ability of int-hpCMPS to trigger in trans RNA degradation of the sensor construct GUS:CMPS. (A) Schematic representation of the sensor construct. A 240 bp part of the CMPS promoter was fused to the 3' of a 1810 bp GUS cDNA unit. The whole construct was under the control of 35S promoter and NOS terminator. (B) Schematic representation of the trigger construct pA-hpCMPS. The 632 bp hpCMPS is not inserted inside an intron and is under the control of 35S promoter and NOS terminator. (C) Co-agroinfiltration of sensor construct GUS:CMPS with empty vector (control, lane 1), int-hpCMPS (lane 2) and pA-hpCMPS (lane 3). Northern analysis showed that only in the case of pA-hpCMPS (lane 3) was a substantial decrease in GUS:CMPS sensor RNA. A GUS 800 bp cDNA fragment was used as probe. The 25S rRNA serves as loading control. (D) Same co-agroinfiltration combination as above. Northern analysis showed that the presence of GFP mRNA of correct size indicated that the int-hpCMPS upon agroinfiltration (lane 2) was correctly spliced. The full GFP cDNA was used as a probe. The 25S rRNA serves as loading control. (E) Same co-agroinfiltration combination as above. siRNA analysis showed 21-nt GUS siRNAs only in the case of pA-hpCMPS (lane 3). These GUS siRNAs are secondary and indicate that only the pA-hpCMPS (lane3) but not the int-hpCMPS (lane 2) is able to lead to in trans RNAi of the sensor construct GUS:CMPS.
SEQ ID NO. 1 shows the third intron of the gene *LeRDR1*;
SEQ ID NO. 2 shows the recombinant GFPint construct;
SEQ ID NO. 3 shows the CMPS promoter sequence;
SEQ ID NO. 26 shows the GUS probe sequence;
SEQ ID NO. 27 shows the GFP5 probe sequence; and
SEQ ID NO. 28 shows the CMPS probe sequence.

SEQ ID NO. 4 to 25 show the primer and probe sequences as used in the examples, and according to the following tables:

**Table 1. Primers used for generation of constructs. In small letters are indicated the mutated sites.**

| **Primer name** | **Sequence from 5' to 3'** |
|---|---|
| GFPPvuII | GATACGTGCAGctgAGGACCATC (SEQ ID NO. 4) |
| GFPMIyI | GATACGgagtcGAGAGGACCATC (SEQ ID NO. 5) |
| IntEco1051 | tacGTAAATTTTACGTTGGAAAC (SEQ ID NO. 6) |
| IntXbaI | GTAAGTTTcTAGAAGGCTTAACC (SEQ ID NO. 7) |
| IntBgIII | CATGAGTCATGAGATcTAAAGGC (SEQ ID NO. 8) |
| IntPvuII | GTGTTAAAACTTTGATGACAGctg (SEQ ID NO. 9) |
| CMPSBglIIF | agaTCTGACGAACAAATAAGA (SEQ ID NO. 10) |
| CMPSBamHIF | GAACAAATAgGATcCGTGGCC (SEQ ID NO. 11) |
| CMPSXbaIR | TTCTAGAGATCCTACTTA (SEQ ID NO. 12) |
| CMPSBglIIR | TTCTAGAGATCTTACTTCTA (SEQ ID NO. 13) |
| FpAtmr | GTaGATCtACGCAGCCGCTTT (SEQ ID NO. 14) |
| RpAtmr | TGTTAGCCTAGGGCTTTA (SEQ ID NO. 15) |
| pTBgIIIF | GCCGCCAAaGATCTGATG (SEQ ID NO. 16) |
| pTPstIR | CGTCCTGCAGTTCATTCA (SEQ ID NO. 17) |
| pTXbaIF | TCTCTAGAAGCTTGTCGA (SEQ ID NO. 18) |
| pTBgIIIR | TGTCCAGATctCCCAGTA (SEQ ID NO. 19) |

**Table 2. Primers used for bisulfite PCR.**

| **Primer name** | **Sequence from 5' to 3'** |
|---|---|
| BisF1 | GTGAAATAATGYGTYTGAYAAAGGTTAG (SEQ ID NO. 20) |
| BisR1 | ACATTTCCCAAACTACCCTTACTTATTTC (SEQ ID NO. 21) |
| BisF2 | GTYAYTAYTTTYTYTTATGGTGTTYAATG (SEQ ID NO. 22) |
| BisR2 | TCTATAAATACTTARCCCCTCCCTCATT (SEQ ID NO. 23) |
| BisF3 | GAATYAATTTGAYTATYTTTGAAAYTA (SEQ ID NO. 24) |
| BisR3 | AATRATTTCCTCCCATAARTTARTRTA (SEQ ID NO. 25) |

### EXAMPLES

### Introduction

A system that enables initiation of nuclear (n) but not cytoplasmatic (c) RNA interference (RNAi), in particular RdDM, requires trigger molecules that are retained in the nucleus and that are exclusively fed into the nuclear RNAi pathway. To fulfil the former requirement, advantage was taken of the fact that intron sequences stay in the nucleus after splicing (Qiau et al., 1992). It was proposed that if an RNAi trigger is inserted into an intron it should be retained in the nucleus as a part of it. However, it was unclear whether such a trigger would impair the splicing process in plant cells. In addition, it was not known whether intronic trigger molecules could be processed by the RdDM machinery.

### Example 1: Generation of the int-hpCMPS construct and introduction into tobacco

As a primary transgene construct carrying the intron the full-length (634 bp) cDNA of intron 3 of the *LeRDR1* (see SEQ ID NO. 1 for sequence data) was inserted into the *mGFP5* cDNA (Haseloff et al., 1997) resulting in the *GFPint* construct (see SEQ ID NO. 2 for the sequence). A hairpin RNA (hp-RNA) was selected as an RNAi trigger for targeting the promoter of a reporter gene. This allowed the study of trans-methylation of the reporter sequence but prevented a possible interference with reporter gene-produced RNA. The hp-RNA construct contained a 240 bp antisense and a 215 bp sense fragment of the *Cestrum yellow leaf curling virus* (CmYLCV) *CMPS* promoter (Stavolone et al., 2003; SEQ ID NO. 3). The sense and anti-sense *CMPS* fragments sharing 214 bp of homology were separated by an inverted 177 bp fragment of the *LeRDR1* intron 3 to produce the hairpin *CMPS* construct (*hpCMPS*). The *hpCMPS* was finally inserted into the intron of *GFPint* (Fig. 1A) giving the *int-hpCMPS* construct. The insertion site of the *hpCMPS* was randomly chosen since plant introns do not have absolute requirements for branch sites and polypyrimidine tracts. An A/T-rich base composition, a minimal size of 70 bp, and the presence of a 5' GT- and 3' AG-splice site were sufficient for intron functionality (Goodall and Filipowicz, 1991).

The *GFPint* and *int-hpCMPS* constructs were cloned into the expression cassette of the pPCV702SM binary vector (Wassenegger et al., 1994). The binary vector derivatives were introduced into *Agrobacterium tumefaciens* that were then used to transform tobacco plants. Independent transformants (SR1-GFPint and SR1-int-hpCMPS) were analyzed for the presence of the T-DNA by PCR. A visual inspection under UV-light revealed that although to varying degrees, the T-DNA-containing SR1-GFPint and SR1-int-hpCMPS plants displayed green fluorescence. The fact that the transgenic plants were fluorescing indicated that the GFP was functional. This in turn suggested that both transgenes were spliced. To further establish, whether the intron was fully and correctly spliced *in planta,* Northern blot analysis was performed. Total RNA extracted from individual SR1-GFPint and SR1-int-hpCMPS lines was hybridized against a *GFP* cDNA probe. All SR1-GFPint plants analyzed produced an RNA with a size that corresponded to the RNA of the GFP-expressing SR1GFP line. The SR1GFP line contained the same *GFP* cDNA as was present in the *GFPint* and *int-hpCMPS* constructs but lacked an intron (Vogt et al., 2004). Similarly, a weak (L1) and a moderate GFP-fluorescing SR1-int-hpCMPS line (H1) displayed a hybridizing RNA with a size of a fully spliced transgene RNA (Fig. 1B, lane 1 and 2). No additional RNA hybridized with the probe demonstrating that the *GFP* pre-mRNA was fully spliced. Finally, Western blot analysis with protein extracts from the SR1-int-hpCMPS lines L1 and H1 was performed. A GFP-specific antibody detected a single 29 kDa protein corresponding to the GFP expressed in the SR1 GFP line (Fig. 1C). In summary, the *LeRDR1* intron 3 proved to be functional and was efficiently and accurately spliced, even though it contained the 632 bp *hpCMPS* fragment.

### Example 2: The int-hpCMPS hp-RNA was processed into 24-nt but not 21-nt siRNAs

Total RNA from the SR1-int-hpCMPS lines L1 and H1 was applied to siRNA analysis. Hybridization of the Northern blot using a *CMPS*-specific probe only revealed a signal with the sample of the H1 line (Fig. 2A, lane 4). The size of the hybridizing RNA corresponded to 24-nt long siRNAs. No 21-nt *CMPS* siRNAs were detectable. In order to achieve a good resolution of small RNAs in tobacco, instead of the routinely analyzed 20-30 µg, only 5 µg of total RNA were loaded onto the gel. Although loading of high RNA concentrations increased the siRNA hybridization signals it resulted in insufficient small RNA separation. Thus, 24-nt siRNAs were only detectable in samples of the SR1-int-hpCMPS line H1 (Figure 2A, lane 4) but not in those of line L1 (Figure 2A, lane 3).

Transgene transcripts can induce the production of 21-nt and 24-nt siRNAs (Wang et al., 2008). However, surprisingly expression of the *int-hpCMPS* transgene construct represents the first example in which only 24-nt but not 21-nt siRNAs accumulated. Agroinfiltration of a GFP hairpin construct in SR1-int-hpCMPS plants produced both sizes of GFP siRNAs (Figure 2B, lane 1) indicating that biosynthesis of 21-nt siRNA was not impaired in SR1-int-hpCMPS lines. Finally, the fact that no *GFP* siRNAs were detectable in non-infiltrated SR1-int-hpCMPS lines demonstrated that GFP silencing was not induced (Fig. 2B, lane 2). This finding was in accordance with the absence of *GFP* mRNA degradation products in SR1-int-hpCMPS lines (Fig. 1).

Surprisingly, the data indicated that processing of the *int-hpCMPS* into siRNAs was exclusively catalyzed by the nRNAi machinery. Moreover, the absence of *GFP* siRNAs pointed out that nRNAi was not associated with transitive silencing. In this context it should be noted that intronic siRNAs were also absent.

### Example 3: Initiation of RdDM in SR1-int-hpCMPS lines

In order to investigate the capacity of the intronic hp-RNA to trigger RdDM of cognate DNA, the methylation status of the int-hpCMPS transgene was examined by Southern blot analysis using the methylation-sensitive restriction endonuclease HaeIII. A single HaeIII restriction site (5'-GGCC-3') was located in the antisense and sense *CMPS* sequences, respectively (Fig. 3A, H(2) and H(3)). Additional HaeIII sites were up- and downstream within the *GFP* sequence (Fig. 3A, H(1) and H(4)). In the internal *CMPS* sequences, cytosines within the HaeIII H(2) and H(3) sites were neither in a CpG nor CpHpG (H = C, T or A) context. Thus, Southern blot analysis of the *CMPS* sequences with HaeIII allowed detection of only asymmetric methylation which is a hallmark of RdDM (Pélissier et al., 1999). In addition to digestion with HaeIII, genomic DNA was pre-cut with the methylation-insensitive restriction endonuclease DraI. Pre-digestion was performed to reduce the average restriction fragment sizes from 10 to 1 kb. No DraI site (5'-TTTAAA-3') was present in the *int-hpCMPS* transgene. As a control for fully cleaved DNA 1 ng the pPCV702SM int-hpCMPS plasmid DNA was mixed with 20 µg of tobacco wild type DNA (Fig. 3B, lane 3). Hybridization with the CMPS-probe (Fig. 3A, black line) revealed three fragments with sizes of 153 bp, 376 bp and 757 bp. The DNA of the SR1-int-hpCMPS line H1 produced three additional hybridizing HaeIII-fragments with sizes of 529 bp, 910 bp and 1286 bp (Fig. 3B, lane1). These fragments can be explained by partial restriction of the HaeIII H(2) and H(3) sites and full cleavage of the HaeIII H(1) and H(4) sites. These data indicate that the H(2) and H(3) sites were partially methylated whereas sites 1 and 4 were virtually free of cytosine methylation.

In order to obtain detailed data on the methylation status of the transgene, bisulfite sequencing was conducted. Treatment of DNA with sodium bisulfite results in conversion of non-methylated but not methylated cytosine to uracile. During PCR amplification DNA polymerases mistake uracile for thymine. Thus, the newly synthesized DNA strand contains adenine instead of guanine at positions complementary to converted cytosines. Sequences of PCR products from bisulfite-treated DNA thus exhibit cytosines for methylated but thymines for non-methylated cytosines. To ensure that cytosines within the sequences of interest could be fully converted, bisulfite sequencing of plasmid DNA was used as a control. Approximately 40 pg of the pPCV702SM int-hpCMPS plasmid DNA were mixed with 2 µg of tobacco wild type DNA to exclude any inhibitory effect of the genomic DNA.

The plasmid/tobacco DNA sample and 2 µg of genomic DNA pooled from the three independent SR1-int-hpCMPS lines L1, M1 and H1 were subjected to bisulfite sequencing. Two transgene fragments were analyzed, the 254 bp CMPS fragment (CMPS₂₅₄) containing a part of the 5' GFP, the 5' intron, the CMPS antisense and the 5' spacer sequences and the 360 bp CMPS fragment (CMPS₃₆₀) containing the 3' spacer, the CMPS sense and a part of the 3' intron sequences (Fig. 4). Bisulfite sequencing is strand-specific. However, the focus was on the analysis of the upper strand using the primer pairs BisF2/BisR2 for CMPS₂₅₄ and BisF3/BisR3 for CMPS₃₆₀ PCR amplification (see Table 1 for primer sequences). Sequencing of cloned PCR products revealed that inserts originated from the pPCV702SM int-hpCMPS plasmid were fully converted confirming that the plasmid DNA was not methylated (Fig. 4, lower line). In contrast, the sense and antisense CMPS sequences amplified from the plant genome-integrated transgene were methylated to almost 100% (Fig. 4, upper line). Importantly, methylation did not spread into sequences flanking the CMPS-specific sequences. All cytosines within the intronic, the GFP and the spacer sequences were found to be converted in all clones (Fig. 4, upper line). In summary, these data demonstrate that an intronic hairpin can efficiently initiate RdDM leading to heavy and specific *de novo* methylation of cognate sequence.

The PCR products obtained from bisulfite treated DNA were not directly sequenced. Only individual clones containing PCR products were analyzed. Thus, the transgene methylation status of single cells was examined. Because in each case only 10 clones were sequenced, no statistically relevant data were presented. To validate, at least in part, the data obtained from bisulfite sequencing, restriction analysis of the PCR products was performed. If a cytosine residue located in a restriction site of the authentic sequence is converted the site will be destroyed. Thus, PCR products amplified from this DNA will lack the site. Analysis of the PCR product will therefore give an overview of the DNA methylation status of the tissue from which the genomic DNA was extracted. Only a limited number of sites can be analyzed. Two parts of the transgene were analyzed by bisulfite. In the 254 bp CMPS₂₅₄, the junction between the 5' intron and the CMPS-specific antisense fragment contained an XbaI site (5'-TCTAGA-3') (Fig. 4). Bisulfite sequencing of the DNA from the SR1-int-hpCMPS plants indicated that this XbaI site was methylated in almost 50% of the leave cells (Fig. 4). Thus, one would expect that the PCR product amplified from this DNA would be cleaved to approximately 50%. Full cleavage of the CMPS₂₅₄ with XbaI results in a 150 and 104 bp fragment (Fig. 5A, lane 2). In accordance with our assumption, about half of the PCR product was digested (Figure 5A, lane 4). In contrast to partial cleavage, the PCR product amplified from bisulfite-treated pPCV702SM int-hpCMPS plasmid DNA was fully cut (Figure 5A, lane 3). This was also in agreement with the bisulfite data presented in Figure 4.

The 360 bp CMPS₃₆₀ was analyzed using HpyCH4IV (5'-ACGT-3'). Full cleavage with HpyCH4IV results in a 129 and a 231 bp fragment (Fig. 5B, lane 2). In the DNA of the SR1-int-hpCMPS lines this site appeared to be methylated to almost 100% (Fig 4). Concordantly, restriction of the PCR product amplified with bisulfite-treated genomic DNA of these lines only produced the 129 and 231 bp fragments, no 360 bp full-length fragment was detectable (Fig 5B, lane 4). As expected, the PCR product of bisulfite-treated plasmid DNA was not cleaved by HpyCH4IV (Figure 5B, lane 3). In summary, Southern blot analysis, bisulfite sequencing and restriction analysis of PCR products amplified from bisulfite-treated DNA clearly demonstrated that the int-hpCMPS construct efficiently initiated RdDM. Methylation was highly specific for the sequences that shared homology with the double-stranded part of the *CMPS* hpRNA. Neither the spacer nor intron sequences flanking the sense and antisense copy of the *CMPS* were targeted by RdDM.

### Example 4: The int-hpCMPS efficiently triggered trans-methylation of a sensor construct

It was demonstrated that the *CMPS* hpRNA induced specific methylation of the genome-integrated transgene region from which the hpRNA was transcribed. In order to investigate whether in addition to this *cis*-methylation, the *int-hpCMPS* had the capacity to trigger *trans-*methylation, a 'methylation-sensor' construct was introduced into SR1 tobacco. The construct comprised a 791 bp *red-shifted GFP* (*rsGFP*) cDNA unit driven by the full-length 406 bp long *CMPS* promoter (*P_{CMPS}-rsGFP*) (Fig. 6A). Seven independent SR1-P_{CMPS}-rsGFP plant lines that were screened for *rsGFP* expression by Northern blot analysis moderately expressed the transgene. No *CMPS* siRNAs were detectable in these lines. The SR1-P_{CMPS}-rsGFP line 1 was genetically crossed with the SR1-int-hpCMPS line H1 to generate the SR1-P_{CMPS}/hpCMPS line 1. Progeny plants of the crossing were grown up and the presence of both transgenes was verified by PCR. The SR1-P_{CMPS}/hpCMPS progeny accumulated CMPS siRNAs to a level that was found in the parental SR1-int-hpCMPS line H1. Again, only 24-nt siRNAs were detectable indicating that the presence of the *P_{CMPS}*/*hpCMPS* had no effect on CMPS siRNA production. In order to investigate whether the presence of the *int-hpCMPS* transgene led to methylation of the *P_{CMPS}* sequence *in trans,* bisulfite sequencing was performed. A 226 bp fragment of the *CMPS* promoter (CMPS₂₂₆) was selected for analysis. It should be noted that only the 3' half of this fragment overlapped with the *hpCMPS* sequence (Fig. 7). DNA from 3 independent progeny plants of SR1-P_{CMPS}/hpCMPS line 1 was pooled, applied to bisulfite treatment and amplified with the primer pair BisF1/BisR1 (see Table 1 for primer sequences). The pPCV800 CMPS-rsGFP plasmid DNA containing the *P_{CMPS}-rsGFP* sequence and genomic DNA from the T1 generation of the SR1-P_{CMPS}-rsGFP line 1 served as controls (Fig. 7). In these controls, the CMPS₂₂₆ was virtually free of methylation (Fig. 7, lower and middle line, respectively), In the SR1-P_{CMPS}/hpCMPS progeny, the 3' half of the CMPS₂₂₆ overlapping with the *hpCMPS* region was fully methylated (Fig. 7, bold sequence). Importantly, the non-overlapping part of the *CMPS* promoter was not methylated demonstrating that spreading of methylation had not taken place in the double transformants. Together our data demonstrated that the intronic *CMPS* hairpin construct triggered extremely efficient and highly specific methylation of the sensor sequence *in trans.*

### Example 5: The int-hpCMPS failed to trigger RNA degradation of a sensor construct

The *int-hpCMPS* construct efficiently triggered *cis-* and *trans*-methylation. However, it was not clear whether the *CMPS* hp-RNA would be able to mediate target RNA cleavage. We could hardly detect CMPS siRNAs from the intronic construct. If detectable they were of 24-nt in size (Fig. 2A, lane 4). However, we could not exclude that the sensitivity of the detection system was too low to detect all the siRNAs produced in the SR1-int-hpCMPS lines, including those of 21-nt class. They could presumably accumulate below the detection limit but would be sufficiently abundant to initiate PTGS. To study if the *int-hpCMPS* PTGS could be initiated, an PTGS sensor construct was generated. The β*-glucuronidase* (*GUS*) cDNA was transcriptionally fused at its 3' end to a 240 bp long *CMPS* promoter fragment. The 240 bp *CMPS* fragment precisely corresponded to the *CMPS* sense copy present the *int-hpCMPS* construct (Fig. 1A). The fusion product (*GUS:CMPS*) was inserted into the expression cassette of the pPCV702SM binary vector (Fig. 7A) which was subsequently introduced into A. *tumefaciens.* As a PTGS trigger control, a CMPS hairpin-containing cassette was produced in which the hpRNA construct was not located in an intron but was flanked by the 35S promoter and the NOS terminator. This was achieved by omitting the GFP and intronic sequences from the original pPCV702SM *int-hpCMPS* cassette (Fig. 7B). The resulting construct, *pA-hpCMPS,* was also introduced into *A. tumefaciens.* Our prediction was that in contrast to the hpRNA produced by the *int-hpCMPS* the hpRNA of the *pA-hpCMPS* would exit the nucleus and would be processed in the cytoplasm to 24 and 21-nt siRNAs, with the latter leading to silencing of the *GUS:CMPS* mRNA.

The *GUS:CMPS* was used for co-agroinfiltration in wild type plants in the following combinations: (i) *GUS:CMPS* + Δ (expression cassette lacking 35S and pA), (ii) *GUS:CMPS* + *int-hpCMPS* and (iii) *GUS:CMPS* + *pA-hpCMPS.* Total RNA was isolated 4 dpi and subjected to RNA analysis. The levels of GUS:CMPS mRNA were dramatically decreased in the case of (iii) when compared to (i) and (ii). The presence of mature GFP mRNA demonstrated that after agroinfiltration the expression and processing of the intronic hairpin was not impaired. Abundant CMPS siRNAs were detected only in the case of pA-hpCMPS and not in the case of int-hpCMPS although the intronic construct was efficiently processed. The data above demonstrated that the *int-hpCMPS* failed to trigger degradation of the sensor mRNA, whereas the *pA-hpCMPS* did. GUS secondary siRNAs were locally produced only after co-agroinfiltration of *GUS:CMPS* with *pA-hpCMPS* but not with *int-hpCMPS.*

## Claims

1. A method for modifying the methylation pattern in a target DNA sequence of a plant cell, comprising the steps of,
a) providing a polynucleotide suitable for the induction of RNA-directed DNA methylation in said plant cell, said polynucleotide comprising a sequence that is complementary to said target DNA sequence of said plant cell and that is inserted into a plant intron,
b) introducing said polynucleotide into said plant cell,
c) expressing said polynucleotide in said transformed cell, thereby inducing RNA-directed DNA methylation, and
d) determining the methylation pattern of said target DNA sequence in said transformed cells of the plant.

2. A method for producing a transgene-free plant with an altered methylation pattern in a target DNA sequence thereof, comprising the steps of,
a) providing a polynucleotide suitable for the induction of RNA-directed DNA methylation in said plant, said polynucleotide comprising a sequence that is complementary to said target DNA sequence,
b) introducing said polynucleotide into cells of a plant,
c) expressing said polynucleotide in said transformed cells, thereby inducing RNA-directed DNA methylation,
d) determining the methylation pattern of said target DNA sequence in said transformed cells of the plant,
e) selecting and self-crossing of a transformed plant that shows an altered methylation pattern in said target DNA sequence, and
f) selecting a plant from the progeny of the cross in e) which is **characterized in that** it is devoid of said polynucleotide as introduced according to step b).

3. The method according to any of claims 1 or 2, wherein said polynucleotide encodes for a dsRNA, preferably a dsRNA-hairpin that is encoded by contiguous sense and anti-sense sequences that are separated by a spacer.

4. The method according to any of claims 2 or 3, wherein said polynucleotide is inserted into a plant intron.

5. The method according to claim 4, wherein said plant intron comprises a five prime GT splice site and a three prime AG splice site, and further has, before insertion of any additional sequences, a final size of at least about 70 base pairs.

6. The method according to any of claims 3 to 5, wherein said polynucleotide is operable linked to a promoter and to a polyadenylation site, wherein said promoter is **characterized in that** it is functional in said cell of said plant.

7. The method according to any of claims 1 to 6, wherein said plant target DNA sequence is an endogenous regulatory sequence that regulates plant DNA transcription, preferably selected from, but not limited to, endogenous promoter sequences, enhancers, silencers, exon-intron boundaries, five prime UTRs, three prime UTRs, or insulators.

8. A method for the production of a transgene-free plant having an altered gene expression profile for a target gene, comprising a method according to any of claims 2 to 7, and g) determining the gene expression for said target gene.

9. Use of a plant produced according to a method according to any of claims 2 to 8 for the production of seeds, fruits, stems, roots and leaves or other plant derived products with reduced expression of a gene, preferably a gene encoding a product that is harmful for animals, humans or plants, preferably genes encoding allergens or genes influencing the level of poisonous biochemical substances in a plant, or a gene encoding an unwanted trait as for example a gene involved in the onset of over-ripeness.

## Patentansprüche

1. Verfahren zur Modifizierung des Methylierungsmusters in einer DNA-Zielsequenz einer Pflanzenzelle, umfassend die Schritte von,
a) zur Verfügung stellen eines Polynukleotids, das zur Induktion von RNA-gerichteter DNA-Methylierung in der Pflanzenzelle geeignet ist, wobei das Polynukleotid eine Sequenz umfasst, die zu der DNA-Zielsequenz der Pflanzenzelle komplementär ist und die in ein pflanzliches Intron insertiert wird,
b) Einführen des Polynukleotids in die Pflanzenzelle,
c) Exprimieren des Polynukleotids in der transformierten Zelle, wodurch RNAgerichtete DNA-Methylierung induziert wird, und
d) Bestimmen des Methylierungsmusters der DNA-Zielsequenz in den transformierten Zellen der Pflanze.

2. Verfahren zur Herstellung einer transgen-freien Pflanze mit einem veränderten Methylierungsmuster in einer DNA-Zielsequenz davon, umfassend die Schritte von,
a) zur Verfügung stellen eines Polynukleotids, das zur Induktion von RNA-gerichteter DNA-Methylierung in der Pflanze geeignet ist, wobei das Polynukleotid eine Sequenz umfasst, die zu der DNA-Zielsequenz der Pflanzenzelle komplementär ist,
b) Einführen des Polynukleotids in Zellen einer Pflanze,
c) Exprimieren des Polynukleotids in der transformierten Zelle, wodurch RNAgerichtete DNA-Methylierung induziert wird,
d) Bestimmen des Methylierungsmusters der DNA-Zielsequenz in den transformierten Zellen der Pflanze,
e) Auswählen und Selbstkreuzung einer transformierten Pflanze, die ein verändertes Methylierungsmuster in der DNA-Zielsequenz zeigt, und
f) Auswählen einer Pflanze aus den Nachkommen der Kreuzung in e), die **dadurch gekennzeichnet ist, dass** ihr das gemäß Schritt b) eingeführte Polynukleotid fehlt.

3. Verfahren nach einem der Ansprüche 1 oder 2, wobei das Polynukleotid für eine dsRNA kodiert, bevorzugt eine dsRNA-Haarnadel, die durch durchgehende sense- und anti-sense-Sequenzen kodiert wird, die durch einen Spacer getrennt sind.

4. Verfahren nach einem der Ansprüche 2 oder 3, wobei das Polynukleotid in ein pflanzliches Intron insertiert wird.

5. Verfahren nach Anspruch 4, wobei das pflanzliche Intron eine fünf-prime GT Splicestelle und eine drei-prime AG Splicestelle umfasst und weiterhin vor der Insertion irgendeiner zusätzlichen Sequenz eine fmale Größe von mindestens ungefähr 70 Basenpaaren hat.

6. Verfahren nach einem der Ansprüche 3 bis 5, wobei das Polynukleotid operabel mit einem Promotor und einer Polyadenylierungsstelle verknüpft ist, wobei der Promotor **dadurch gekennzeichnet ist, dass** er in der Zelle der Pflanze funktional ist.

7. Verfahren nach einem der Ansprüche 1 bis 6, wobei die pflanzliche DNA-Zielsequenz eine endogene regulatorische Sequenz ist, die die pflanzliche DNA-Transkription reguliert, bevorzugt ausgewählt aus, jedoch nicht beschränkt auf, endogene Promotorsequenzen, Enhancern, Silencern, Exon-Intron Grenzen, fünf-prime UTRs, drei-prime UTRs oder Isolatoren.

8. Verfahren zur Herstellung einer transgen-freien Pflanze, die ein verändertes Genexpressionsprofil für ein Zielgen aufweist, umfassend ein Verfahren nach einem der Ansprüche 2 bis 7 und g) Bestimmen der Genexpression für das Zielgen.

9. Verwendung eine Pflanze, hergestellt gemäß einem Verfahren nach einem der Ansprüche 2 bis 8 zur Herstellung von Samen, Früchten, dem Spross, Wurzeln und Blättern oder anderen Pflanzen-abgeleiteten Produkten mit verringerter Expression eines Gens, bevorzugt eines Gens, das für ein Produkt kodiert, das für Tiere, Menschen oder Pflanzen schädlich ist, bevorzugt Gene, die für Allergene kodieren, oder Gene, die den Spiegel von giftigen biochemischen Substanzen in einer Pflanze beeinflussen, oder ein Gen, das für einen unerwünschten Marker kodiert, wie zum Beispiel ein Gen, das an der Entstehung von Überreife beteiligt ist.

## Revendications

1. Procédé pour la modification du schéma de méthylation dans une séquence d'ADN cible d'une cellule végétale, comprenant les étapes suivantes :
a) la fourniture d'un polynucléotide adapté à l'induction de la méthylation de l'ADN dirigée par l'ARN dans ladite cellule végétale, ledit polynucléotide comprenant une séquence qui est complémentaire à ladite séquence d'ADN cible de ladite cellule végétale et qui est insérée dans un intron végétal,
b) l'introduction dudit polynucléotide dans ladite cellule végétale,
c) l'expression dudit polynucléotide dans ladite cellule transformée, induisant ainsi la méthylation de l'ADN dirigée par l'ARN, et
d) la détermination du schéma de méthylation de ladite séquence d'ADN cible dans lesdites cellules transformées de la plante.

2. Procédé pour la production d'une plante dépourvue de transgène et présentant un schéma de méthylation modifié dans une séquence d'ADN cible de cette dernière, comprenant les étapes suivantes :
a) la fourniture d'un polynucléotide adapté à l'induction de la méthylation de l'ADN dirigée par l'ARN dans ladite plante, ledit polynucléotide comprenant une séquence qui est complémentaire à ladite séquence d'ADN cible,
b) l'introduction dudit polynucléotide dans les cellules d'une plante,
c) l'expression dudit polynucléotide dans lesdites cellules transformées, induisant ainsi la méthylation de l'ADN dirigée par l'ARN,
d) la détermination du schéma de méthylation de ladite séquence d'ADN cible dans lesdites cellules transformées de la plante,
e) la sélection et l'auto-croisement d'une plante transformée qui présente un schéma de méthylation modifié dans ladite séquence d'ADN cible, et
f) la sélection d'une plante parmi celles obtenues du croisement exposé en e) qui est **caractérisée par le fait qu'**elle est dépourvue dudit polynucléotide tel qu'introduit conformément à l'étape b).

3. Procédé conforme à la revendication 1 ou 2, dans lequel ledit polynucléotide code pour un ARNdb, de préférence un ARNdb en épingle à cheveux codé par des séquences sens et antisens contiguës qui sont séparées par un espaceur.

4. Procédé conforme à la revendication 2 ou 3, dans lequel ledit polynucléotide est inséré dans un intron végétal.

5. Procédé conforme à la revendication 4, dans lequel ledit intron végétal comprend un site d'épissage GT en 5' et un site d'épissage AG en 3', et présente également, avant l'insertion d'éventuelles séquences supplémentaires, une taille finale d'au moins 70 paires de bases environ.

6. Procédé conforme à l'une des revendications 3 à 5, dans lequel ledit polynucléotide est lié de façon opérable à un promoteur et à un site de polyadénylation, dans lequel ledit promoteur est **caractérisé par le fait qu'**il est fonctionnel dans ladite cellule de ladite plante.

7. Procédé conforme à l'une des revendications 1 à 6, dans lequel ladite séquence d'ADN cible végétale est une séquence régulatrice endogène qui régule la transcription de l'ADN végétal, de préférence sélectionnée à partir de, mais non limitée à, des séquences promotrices endogènes, des amplificateurs, des silenceurs, des frontières exon-intron, des régions non traduites en 5', des régions non traduites en 3', ou des isolants.

8. Procédé pour la production d'une plante dépourvue de transgène présentant un profil d'expression génique modifié pour un gène cible, comprenant un procédé conforme à l'une des revendications 2 à 7, et g) la détermination de l'expression génique pour ledit gène cible.

9. Utilisation d'une plante produite selon un procédé conforme à l'une des revendications 2 à 8 pour la production de graines, fruits, pédoncules, racines et feuilles ou autres produits d'origine végétale avec une expression réduite d'un gène, de préférence un gène codant pour un produit dangereux pour les animaux, les hommes ou les plantes, de préférence des gènes codant pour des allergènes ou des gènes influençant le niveau de substances biochimiques toxiques d'une plante, ou un gène codant pour une caractéristique non souhaitée, comme par exemple un gène impliqué dans la surmaturation.


### SEQUENCE LISTING

<110> Johannes-Gutenberg Universitaet Mainz
<120> METHOD FOR THE PRODUCTION OF A TRANSGENE-FREE PLANT WITH ALTERED METHYLATION PATTERN
<130> FB20591
<160> 28
<170> PatentIn version 3.5
<210> 1
   <211> 635
   <212> DNA
   <213> Nicotiana tabacum
<400> 1
   gtaaatttta cgttggaaac ctattaactg tttacttgta agtttataga aggcttaacc        60
   atttcttgcc tggtctttta ctaatgctct aatccaaatt agtttcaaag atagtcaaat       120
   tgattctcgg gaagcaaaaa gcgacagagg gagtaattga ctgtctttcc ttaattagtt       180
   aagattcact tgtataatat ccccttctca tgagtcatga gatctaaagg catattgaaa       240
   tacaaagaag tcttttcttc cttcccatat tctacactaa cttatgggag gaaatcattt       300
   tcctactggc tacgcaatta ttcaacttgt aactctccag actaggtatg tatttcaact       360
   gcctccctac ctctgaggca gaggtaattt gcgtacactc tactctcctc agacctcact       420
   ttgtggtatc ttactgggta tgtttttgtt gtataattca tcagtacttc atctatctgg       480
   aatattctca acttttgttc atggaagaat gtgttaactt tattaattgt tctttctagt     540
   aaaagtgtaa aactagcttc cacccttgtc aacgctttct atctttaatt cttttttatg     600
   gcattgttac tcaagtgtta aaactttgat gacag                                  635
<210> 2
   <211> 1433
   <212> DNA
   <213> synthetic
<400> 2
   atgaagacta atctttttct ctttctcatc ttttcacttc tcctatcatt atcctcggcc 60
   gaattcagta aaggagaaga acttttcact ggagttgtcc caattcttgt tgaattagat 120
   ggtgatgtta atgggtacaa attttctgtc agtggagagg gtgaaggtga tgcaacatac 180
   ggaaaactta cccttaaatt tatttgcact actggaaaac tacctgttcc atggccaaca 240
   cttgtcacta ctttctctta tggtgttcaa tgcttttcaa gatacccaga tcatatgaag 300
   cggcacgact tcttcaagag cgccatgcct gagggatacg tgcaggtaaa ttttacgttg 360
   gaaacctatt aactgtttac ttgtaagttt ctagaaggct taaccatttc ttgcctggtc 420
   ttttactaat gctctaatcc aaattagttt caaagatagt caaattgatt ctcgggaagc 480
   aaaaagcgac agagggagta attgactgtc tttccttaat tagttaagat tcgcttgtat 540
   aatatcccct tctcatgagt catgagatct aaaggcatat tgaaatacaa agaagtcttt 600
   tcttccttcc catattctac actaacttat gggaggaaat cattttccta ctggctacgc 660
   aattattcaa cttgtaactc tccagactag gtatgtattt caactgcctc cctacctctg 720
   aggcagaggt aatttgcgta cactctactc tcctcagacc tcactttgtg gtatcttact 780
   gggtatgttt ttgttgtata attcatcagt acttcatcta tctggaatat tctcaacttt 840
   tgttcatgga agaatgtgtt aactttatta attgttcttt ctagtaaaag tgtaaaacta 900
   gcttccaccc ttgtcaacgc tttctatctt taattctttt ttatggcatt gttactcaag 960
   tgttaaaact ttgatgacag gagaggacca tcttcttcaa ggacgacggg aactacaaga 1020
   cacgtgctga agtcaagttt gagggagaca ccctcgtcaa caggatcgag cttaagggaa 1080
   tcgatttcaa ggaggacgga aacatcctcg gccacaagtt ggaatacaac tacaactccc 1140
   acaacgtata catcatggcc gacaagcaaa agaacggcat caaagccaac ttcaagaccc 1200
   gccacaacat cgaagacggc ggcgtgcaac tcgctgatca ttatcaacaa aatactccaa 1260
   ttggcgatgg ccctgtcctt ttaccagaca accattacct gtccacacaa tctgcccttt 1320
   cgaaagatcc caacgaaaag agagaccaca tggtccttct tgagtttgta acagctgctg 1380
   ggattacaca tggcatggat gaactataca aacatgatga gctcaatcac tag 1433
<210> 3
   <211> 408
   <212> DNA
   <213> Cestrum yellow leaf curling virus
<400> 3
   gatcctggca gacaaagtgg cagacatact gtcccacaaa tgaagatgga atctgtaaaa 60
   gaaaacgcgt gaaataatgc gtctgacaaa ggttaggtcg gctgccttta atcaatacca 120
   aagtggtccc taccacgatg gaaaaactgt gcagtcggtt tggctttttc tgacgaacaa 180
   ataagattcg tggccgacag gtgggggtcc accatgtgaa ggcatcttca gactccaata 240
   atggagcaat gacgtaaggg cttacgaaat aagtaagggt agtttgggaa atgtccactc 300
   acccgtcagt ctataaatac ttagcccctc cctcattgtt aagggagcaa aatctcagag 360
   agatagtcct agagagagaa agagagcaag tagcctagaa gtaggatc 408
<210> 4
   <211> 23
   <212> DNA
   <213> synthetic
<400> 4
   gatacgtgca gctgaggacc atc 23
<210> 5
   <211> 23
   <212> DNA
   <213> synthetic
<400> 5
   gatacggagt cgagaggacc atc 23
<210> 6
   <211> 23
   <212> DNA
   <213> synthetic
<400> 6
   tacgtaaatt ttacgttgga aac 23
<210> 7
   <211> 23
   <212> DNA
   <213> synthetic
<400> 7
   gtaagtttct agaaggctta acc 23
<210> 8
   <211> 23
   <212> DNA
   <213> synthetic
<400> 8
   catgagtcat gagatctaaa ggc 23
<210> 9
   <211> 24
   <212> DNA
   <213> synthetic
<400> 9
   gtgttaaaac tttgatgaca gctg 24
<210> 10
   <211> 21
   <212> DNA
   <213> synthetic
<400> 10
   agatctgacg aacaaataag a 21
<210> 11
   <211> 21
   <212> DNA
   <213> synthetic
<400> 11
   gaacaaatag gatccgtggc c 21
<210> 12
   <211> 18
   <212> DNA
   <213> synthetic
<400> 12
   ttctagagat cctactta 18
<210> 13
   <211> 20
   <212> DNA
   <213> synthetic
<400> 13
   ttctagagat cttacttcta 20
<210> 14
   <211> 21
   <212> DNA
   <213> synthetic
<400> 14
   gtagatctac gcagccgctt t 21
<210> 15
   <211> 18
   <212> DNA
   <213> synthetic
<400> 15
   tgttagccta gggcttta 18
<210> 16
   <211> 18
   <212> DNA
   <213> synthetic
<400> 16
   gccgccaaag atctgatg 18
<210> 17
   <211> 18
   <212> DNA
   <213> synthetic
<400> 17
   cgtcctgcag ttcattca 18
<210> 18
   <211> 18
   <212> DNA
   <213> synthetic
<400> 18
   tctctagaag cttgtcga 18
<210> 19
   <211> 18
   <212> DNA
   <213> synthetic
<400> 19
   tgtccagatc tcccagta 18
<210> 20
   <211> 28
   <212> DNA
   <213> synthetic
<400> 20
   gtgaaataat gygtytgaya aaggttag 28
<210> 21
   <211> 29
   <212> DNA
   <213> synthetic
<400> 21
   acatttccca aactaccctt acttatttc 29
<210> 22
   <211> 29
   <212> DNA
   <213> synthetic
<400> 22
   gtyaytaytt tytyttatgg tgttyaatg 29
<210> 23
   <211> 28
   <212> DNA
   <213> synthetic
<400> 23
   tctataaata cttarcccct ccctcatt 28
<210> 24
   <211> 27
   <212> DNA
   <213> synthetic
<400> 24
   gaatyaattt gaytatyttt gaaayta 27
<210> 25
   <211> 27
   <212> DNA
   <213> synthetic
<400> 25
   aatratttcc tcccataart tartrta 27
<210> 26
   <211> 963
   <212> DNA
   <213> synthetic
<400> 26
   tgtgggcatt cagtctggat cgcgaaaact gtggaattga tcagcgttgg tgggaaagcg      60
   cgttacaaga aagccgggca attgctgtgc caggcagttt taacgatcag ttcgccgatg     120
   cagatattcg taattatgcg ggcaacgtct ggtatcagcg cgaagtcttt ataccgaaag     180
   gttgggcagg ccagcgtatc gtgctgcgtt tcgatgcggt cactcattac ggcaaagtgt     240
   gggtcaataa tcaggaagtg atggagcatc agggcggcta tacgccattt gaagccgatg     300
   tcacgccgta tgttattgcc gggaaaagtg tacgtatcac cgtttgtgtg aacaacgaac     360
   tgaactggca gactatcccg ccgggaatgg tgattaccga cgaaaacggc aagaaaaagc     420
   agtcttactt ccatgatttc tttaactatg ccggaatcca tcgcagcgta atgctctaca     480
   ccacgccgaa cacctgggtg gacgatatca ccgtggtgac gcatgtcgcg caagactgta     540
   accacgcgtc tgttgactgg caggtggtgg ccaatggtga tgtcagcgtt gaactgcgtg     600
   atgcggatca acaggtggtt gcaactggac aaggcactag cgggactttg caagtggtga     660
   atccgcacct ctggcaaccg ggtgaaggtt atctctatga actgtgcgtc acagccaaaa     720
   gccagacaga gtgtgatatc tacccgcttc gcgtcggcat ccggtcagtg gcagtgaagg     780
   gcgaacagtt cctgattaac cacaaaccgt tctactttac tggctttggt cgtcatgaag     840
   atgcggactt gcgtggcaaa ggattcgata acgtgctgat ggtgcacgac cacgcattaa     900
   tggactggat tggggccaac tcctaccgta cctcgcatta cccttacgct gaagagatgc     960
   tcg                                                                   963
<210> 27
   <211> 792
   <212> DNA
   <213> synthetic
<400> 27
   atgaagacta atctttttct ctttctcatc ttttcacttc tcctatcatt atcctcggcc      60
   gaattcagta aaggagaaga acttttcact ggagttgtcc caattcttgt tgaattagat     120
   ggtgatgtta atgggtacaa attttctgtc agtggagagg gtgaaggtga tgcaacatac     180
   ggaaaactta cccttaaatt tatttgcact actggaaaac tacctgttcc atggccaaca     240
   cttgtcacta ctttctctta tggtgttcaa tgcttttcaa gatacccaga tcatatgaag     300
   cggcacgact tcttcaagag cgccatgcct gagggatacg tgcaggagag gaccatcttc     360
   ttcaaggacg acgggaacta caagacacgt gctgaagtca agtttgaggg agacaccctc     420
   gtcaacagga tcgagcttaa gggaatcgat ttcaaggagg acggaaacat cctcggccac     480
   aagttggaat acaactacaa ctcccacaac gtatacatca tggccgacaa gcaaaagaac     540
   ggcatcaaag ccaacttcaa gacccgccac aacatcgaag acggcggcgt gcaactcgct     600
   gatcattatc aacaaaatac tccaattggc gatggccctg tccttttacc agacaaccat     660
   tacctgtcca cacaatctgc cctttcgaaa gatcccaacg aaaagagaga ccacatggtc     720
   cttcttgagt ttgtaacagc tgctgggatt acacatggca tggatgaact atacaaacat     780
   gatgagcttt aa                                                         792
<210> 28
   <211> 590
   <212> DNA
   <213> Cestrum yellow leaf curling virus
<400> 28
   tctagagatc ctacttctag gctacttgct ctctttctct ctctaggact atctctctga      60
   gattttgctc ccttaacaat gagggagggg ctaagtattt atagactgac gggtgagtgg     120
   acatttccca aactaccctt acttatttcg taagccctta cgtcattgct ccattattgg     180
   agtctgaaga tgccttcaca tggtggaccc ccacctgtcg gccacgaatc ttatttgttc     240
   gtcagatcct aactaattaa ggaaagacag tcaattactc cctctgtcgc tttttgcttc     300
   ccgagaatca atttgactat ctttgaaact aatttggatt agagcattag taaaagacca     360
   ggcaagatcc gtggccgaca ggtgggggtc caccatgtga aggcatcttc agactccaat     420
   aatggagcaa tgacgtaagg gcttacgaaa taagtaaggg tagtttggga aatgtccact     480
   cacccgtcag tctataaata cttagcccct ccctcattgt taagggagca aaatctcaga     540
   gagatagtcc tagagagaga aagagagcaa gtagcctaga agtaagatct                590